# EUROPEAN PATENT APPLICATION

(11) **EP 4 834 873 A1**
(43) Date of publication of application: **04.11.2026**
(21) Application number: 25173202.0
(22) Date of filing: 29.04.2025
(51) Int. Cl.: C07C 33/14

(54) **IMPROVEMENTS IN OR RELATING TO ORGANIC FRAGRANCES**

(71) Applicant: Givaudan SA, 1214 Vernier (CH)
(72) Inventor: The designation of the inventor has not yet been filed
(74) Representative: Global Patents

(57) **Abstract**

A perfume mixture comprising a compound according to formula I or formula II 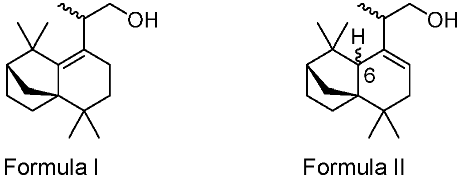 and a method to provide such a mixture.

## Description

### Field of the invention

The present invention relates generally to novel perfume mixtures, methods of making them, and their use in consumer products.

### Sequence listing

The instant application contains a Sequence Listing which has been submitted electronically in XML format, compliant with WIPO Standard ST.26, and is hereby incorporated by reference in its entirety. Said XML copy, created on 31 March 2025, is named 31536.xml and is 13437 bytes in size.

### Background of the invention

Perfume ingredients capable of delivering ambery odour notes to consumer products are important materials in a perfumer's toolbox. They add depth and warmth to fragrances, and as such they are often used as base notes in perfumery to connote a feeling of warmth, sophistication, luxury and elegance.

Ambermax^{™} perfume ingredient is well-known in the perfumery arts. When used in fine and functional perfumery it is able to deliver a powerful, fusing and substantive rich ambery note with some woody cedarwood facets. Its outstanding fabric substantivity makes it a key building block for fabric care fragrances beating all benchmarks on dry cloth. It is used widely in household care, laundry care, and personal care products, as well as in fine perfumery, to generate pleasant odours or if necessary, to mask unpleasant odours.

Ambermax^{™} perfume ingredient was first described in United States Patent US 7,550,417. Only a cursory inspection of the molecule's structure informs the skilled addressee of its several stereocenters and positional isomers. Indeed, the commercial form of the ingredient exists as an elaborate mixture of its stereo-, regio-, constitutional and conformational isomers.

The fact that perfume ingredients, such as Ambermax^{™}, can exist in the form of complex mixtures of isomers is problematic for the modern perfumer concerned with the sustainable use of precious resources, if only some of the isomers are contributing significantly to the odour of the ingredient, or indeed if others are actually contributing undesirable off-notes.

There remains a need to provide novel perfume mixtures that can impart the same or substantially similar odour note of the commercial ingredient Ambermax^{™}, but which mixtures represent a more efficient use of carbon.

### Summary of the invention

In addressing the problem of providing more sustainable use of perfumery ingredients, the applicant undertook a study of the activity of perfume ingredients at the receptor level using more discriminating and sensitive olfactory receptor assay technology in order to understand more clearly the contribution that the various isomers of complex perfume ingredients play in both their odour character and intensity. Indeed, in the case of the perfume ingredient Ambermax^{™}, the applicant discovered that only very few isomers contribute to the character and intensity of the ingredient, others being only weakly odiferous or even exhibiting undesirable off-notes. This insight has enabled the applicant to propose novel perfume mixtures enriched in isomers that strongly contribute to odour character and intensity of the commercial Ambermax^{™} perfume ingredient, but which are free or substantially free of any isomers, and indeed side-products that do not contribute, or are detrimental, to the desired odour character and intensity.

Accordingly, in a first aspect of the invention there is provided a perfume mixture comprising a compound according to formula I and formula II 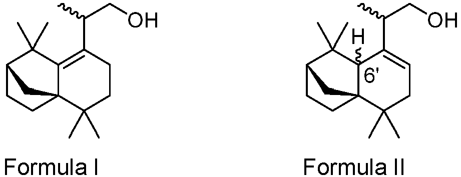 wherein the perfume mixture is free, or substantially free of a compound according to formula III 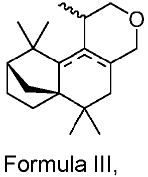 and wherein the wavy bond in the compound of formula I, II and/or III indicates an unspecified configuration at the attached carbon atom, respectively, and the dotted lines together with the solid lines 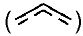 in the compound of formula III indicate one single and one double bond.

In a second aspect of the invention there is provided a perfume composition comprising the perfume mixture of the first aspect of the invention and at least one other perfume ingredient.

In a third aspect of the invention there is provided a consumer product comprising a perfume mixture according to the first aspect of the invention.

In a fourth aspect of the invention there is provided a method of preparing the perfume mixture according to the first aspect of the invention.

The details, examples and preferences provided in relation to any one or more of the stated aspects of the present invention will be further described herein and apply equally to all aspects of the present invention. Any combination of embodiments, examples and preferences described herein below in all possible variations thereof are encompassed by the present invention unless otherwise indicated herein, or otherwise clearly contradicted by context.

### Detailed description of the invention

The first aspect of the invention is concerned with a perfume mixture comprising a compound according to formula I and formula II 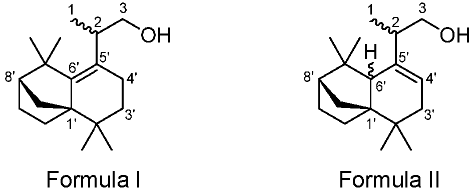 wherein the perfume mixture is free, or substantially free of a compound according to formula IIIa and/or Illb 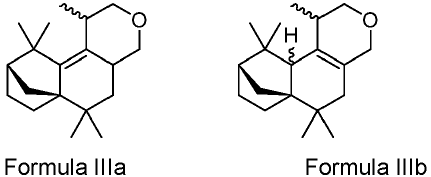 and wherein the wavy bond in the compound of formula I, II, Illa and/or Illb indicate an unspecified configuration at the attached carbon atom, respectively.

To the applicant's knowledge, the preparation and characterization of this isomeric mixture, and its use in perfumery, is not known in the art.

The perfume mixture, including all of its particular embodiments described herein below, was found to possess the advantageous odour properties of commercial grade of the Ambermax^{™} ingredient, as assessed by trained panelists with normal olfactive acuity, however, the perfume mixture represents a far more efficient use of carbon compared to the commercial grade ingredient owing to it being enriched in certain desirable isomers and being free or substantially free of isomers that lack the requisite odour character or intensity.

In particular embodiments of the invention there is provided a perfume mixture comprising the compound according to formula I and the compound according to formula II, wherein the ratio of the compound of formula (I) to the compound of formula (II) is between 1.3 to 2.5, or between 1.5 to 2.0, or between 1.7 to 1.9.

In particular embodiments of the invention the perfume mixture comprises the compound according to formula I in 50-70% by weight, and the compound according to formula II in 28-38% by weight.

As used herein, the term "substantially free" means that to the extent that an isomer is present in a perfume mixture, then it is present in trace amounts below a level that would be consistent with its customary use in perfumery as practiced by skilled perfumers.

In particular, the applicant found that the tetracyclic ethers according to the formula Illa and/or Illb, which are reported in US 7,550,417 to be present in amounts up to 20 wt % in the ingredient Ambermax^{™} do not contribute significantly or at all to the odour character or intensity of the ingredient.

More particularly, its presence would be less than 0.1 wt %, based on the total weight of the perfume mixture.

In embodiments of the present invention, the tetracyclic ethers of formula Illa and/or Illb are present, if at all, at levels of 0.1 wt % or less.

In particular embodiments of the invention there is provided a perfume mixture comprising the compound according to formula I in 50-70% by weight, the compound according to formula II in 28-38% by weight, the compound of formula IIIa in 0.1 wt % or less, and the compound of formula Illb in 0.1 wt % or less.

In a particular embodiment of the invention the perfume mixture is free or substantially free of side-products that either do not contribute or interfere with the desired olfactory character or intensity of the perfume mixture. More particularly, the perfume mixture is free or substantially free of a compound according to formula IV or formula V 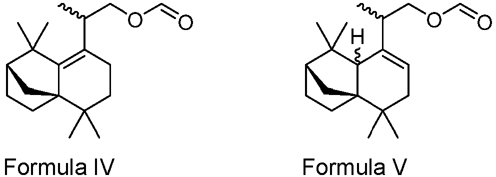 wherein the wavy bond in the compound of formula IV and/or V indicates an unspecified configuration at the attached carbon atom, respectively.

In more particular embodiments of the invention, the compounds of formula IV and V are present, if at all, at levels below 0.5 wt % of the mixture, and more particularly at levels of about 0.3 wt % or below.

In particular embodiments of the invention there is provided a perfume mixture comprising the compound according to formula I in 50-70% by weight, the compound according to formula II in 28-38% by weight, and the compound of formula IV in 0.5 wt % or less, and the compound of formula V in 0.1 wt % or less.

In particular embodiments of the invention there is provided a perfume mixture comprising the compound according to formula I in 50-70% by weight, the compound according to formula II in 28-38% by weight, the compound of formula IIIa in 0.1 wt % or less, the compound of formula Illb in 0.1 wt % or less, the compound of formula IV in 0.5 wt % or less, and the compound of formula V in 0.1 wt % or less.

Applicant found that the compounds of formula I and II which are drivers of odour character and intensity are in the absolute (8'S) configuration, while their respective enantiomers in the (8'R) absolute configuration are less potent.

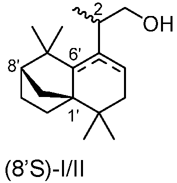

In the compound (8'S)-I/II, the wavy bond indicates an unspecified configuration at the attached carbon atom, respectively, and the dotted lines together with the solid lines indicate one single and one double bond.

In particular, compound of formula I with absolute configuration (1'R, 2S, 8'S) was found to have a 27700x lower odour detection threshold and is therefore significantly more potent compared to its enantiomer of absolute configuration (1'S, 2R, 8'R).

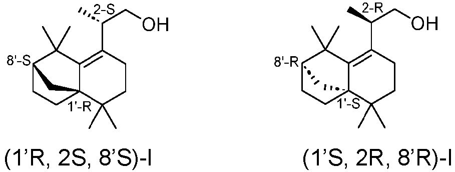

The enantiomers of the compound of formula I are shown in their absolute configuration.

Accordingly, in a particular embodiment of the perfume mixture comprising the compound of the formula I, the enantiomer in the configuration (1'R,2S,8'S) is in an enantiomeric excess, for example of about 20% ee or above, for example of about 40% ee or above, or of about 60 % ee or above.

In particular embodiments of the invention, the compound of formula II comprises the isomeric pair 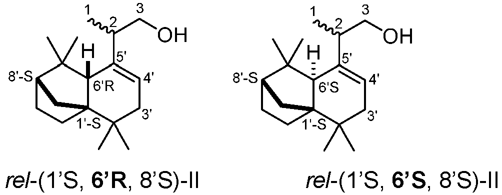 in which the *rel*-(1'S, **6'R,** 8'S) isomer is enriched relative to the *rel*-(1'S, **6'S,** 8'S) isomer. The isomer with hydrogen at position 6' and bridged-byclic system (1', 8') pointing in the same direction is major. In the representation with bold or dashed but unwedged bonds, the relative configuration of hydrogen at position 6' and bridged-byclic system (1', 8') is depicted. Such a representation does not indicate an absolute configuration.

In more particular embodiments, the ratio of *rel*-(1'S, 6'R, 8'S) isomer to the *rel*-(1'S, 6'S, 8'S) isomer of the compound of formula II is at least 9:1.

The applicant has found that perfume mixtures comprising a compound according to formula I and formula II which are diastereomerically enriched in the 8'S isomer of the compound of formula II display a more desirable character and intensity compared with those having higher amounts of the 8'R isomer, and as such, such perfume mixtures represent a more efficient use of carbon and a more sustainable material compared with the commercial Ambermax ^{™} ingredient.

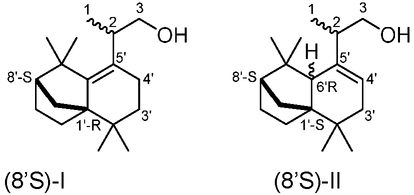

The compounds of formula I and II are shown in their absolute (8'S) configuration.

It should be noted that for the compounds of formula (I) and (II), the absolute configuration of C1 changes in formula I vs formula II due to nomenclature rules, while the arrangement of the substituents remains unchanged.

In particular, compound of formula II with absolute configuration (1'S, 2S, 6'R, 8'S) was found to have a 13400x lower odour detection threshold and is therefore significantly more potent compared to its enantiomer of absolute configuration (1'R, 2R, 6'S, 8'R).

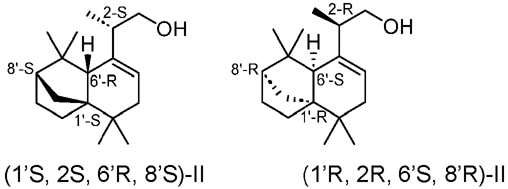

Accordingly, in particular embodiments of the perfume mixture comprising the compound of formula II, the enantiomer in the configuration (1'S 2S, 6'R, 8'S) is in an enantiomeric excess, for example of about 20% ee or above, for example of about 40% ee or above, or of about 60% ee or above.

The impact of the compounds on the overall odour impression is demonstrated by the comparison of the odour detection thresholds. The compounds listed below were obtained by a combination of semi-preparative non-chiral and chiral HPLC and the concentration of the HPLC isolates was determined by chiral GC-FID with external calibration (see example 2).

The determination of the odour detection thresholds required samples of high analytical and olfactory purity. Therefore, special care was taken to prevent trace contaminations of potent stereoisomers influencing the perception of weaker ones and the olfactory purity of each HPLC isolate was verified by chiral GC sniffing technique. If the main peak in the HPLC isolate was odorless during the olfactory purity assessment, the concentration of the stock solution was used to determine the GC odor detection threshold (GTH) and the value was reported as greater than (GTH > x ng). For all compounds where the olfactory purity of the sample could be confirmed by chiral GC sniffing analysis and the main constituent was the sample odor-vector, the GTH determinations were performed by non-chiral gas chromatography-olfactometry as described in Flachsmann et al. (2024).

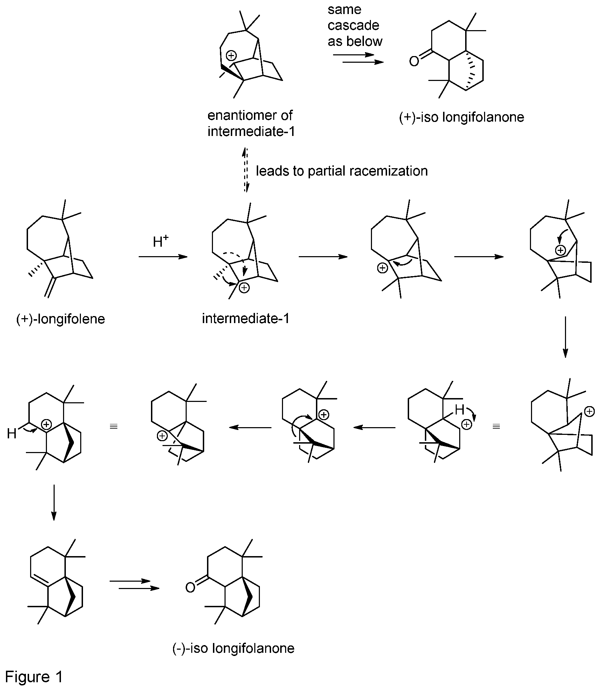

The structure of the individual compounds I and II (including the relative stereochemistry) was assigned based on the NMR data. Each peak could be separated on chiral phase into major and minor enantiomers. Assignment of the absolute stereochemistry is based on the observation that isolongifolanone is enriched to varying degrees in the (-)-enantiomer but never the (+)-enantiomer. This relates back to its preparation from naturally occurring (+)-longifolene, which is converted through a series of cationic rearrangements and follow-up steps (shown below) into (-)-isolongifolanone (Figure 1). Depending on acid strength and conditions, after the first protonation, the intermediate cation "intermediate-1" can partially racemize by Wagner-Meerwein shift into its own enantiomer "enantiomer of intermediate-1", ultimately leading to (+)-isolongifolanone. This explains the varying levels of racemization (and therefore varying ee's) of isolongifolanone, but scalemic mixtures are always enriched in the (-)-isolongifolanone (shown below) because racemization of intermediate-1 can at worst lead to a racemic mixture, without ever inverting the absolute stereochemistry. Based on this rationale, the major enantiomer of each peak was assigned the absolute stereochemistry derived from (-)-isolongifolanone while the minor was assigned the absolute stereochemistry derived from (+)-isolongifolanone.

Furthermore, the activation of the amber receptor OR7C1 by different ambermax isomers was investigated. It was shown that only two compounds are significantly activating the receptor (see example 3). A perfume mixture enriched in those impactful isomers is more powerful and efficient in delivering the desired odour. It is also more sustainable, as it has a higher odour per carbon ratio in comparison with perfume mixture comprising significant amounts of odourless or less impactful isomers.

In the second aspect of the invention there is provided a perfume composition comprising the perfume mixture as described herein and at least one other perfumery ingredient.

The at least one other perfumery ingredient can be a perfumery raw material or it can be a functional ingredient used in perfumery, such as a solvent or the like. The at least one other perfumery ingredient does not include the ingredient Ambermax ^{™}.

In particular embodiments of the invention, the at least one other perfumery ingredient is a solvent for the perfume mixture. The solvent can be selected from any solvent used in the context of perfumery. In particular, the solvent is selected from triethyl citrate and Dowanol Tpm Glycol Ether (Tripropylene Glycol Methyl Ether)

In particular embodiments of the invention, the at least one other perfumery ingredient is a perfume raw material. As stated herein above, the character of the ingredient Ambermax ^{™} is such that it is able to deliver a rich ambery note with some woody cedarwood facets. Perfume raw materials that can be combined with and complement the odour notes of Ambermax ^{™} can be used in a similar manner with perfume mixtures according to the invention. Such perfume raw materials include but are not limited to:
- Ambery materials, for example Ambrofix (3a,6,6,9a-Tetramethyldodecahydronaphtho[2,1-B]furan), Amberketal (3,8,8,11a-Tetramethyldodecahydro-5h-3,5a-epoxy-napht(2,1-C)oxepin, Amber Xtreme (2h-Indeno[4,5-B]furan, decahydro-2,2,6,6,7,8,8-heptamethyl-), Ambrocenide (4h-4a,9-Methanoazuleno[5,6-D]-1,3-dioxole,octahydro-2,2,5,8), Nimberol (Cyclohexanepropanol, 2,2,6-Trimethyl-Alpha-Propyl-).
- Woody materials, for example Cashmeran (6,7-dihydro-1,1,2,3,3-pentamethyl-4(5h)-indanone), Iso E Super (2-acetyl-1,2,3,4,5,6,7,8-octahydro-2,3,8,8-tetra-methylnaphtalene), Georgywood (2-acetyl-1,2,3,4,5,6,7,8-octahydro-1,2,8,8-tetramethylnaphthalene and 2-acetyl-1,2,3,4,5,6,7,8- octahydro-2,3,8,8-tetramethylnaphthalene), Evernyl (methyl 2,4-dihydroxy-3,6-dimethylbenzoate), Patchouli oil, Cedryl methyl ether, Radjanol (2-ethyl-4-(2,2,3-trimethyl-3-cyclopenten-1-yl)-2-buten-1-ol), Javanol ((1-methyl-2-(1,2,2-trimethylbicyclo(3.1.0)-hex-3-ylmethyl)cyclopropyl)methanol, Polysantol (trans-3,3-dimethyl-5-(2,2,3-trimethyl-cyclopent-3-en-1-yl)-pent-4-en-2-ol).
- Musky materials, for example Galaxolide (1,3,4,6,7,8-hexahydro-4,6,6,7,8,8-hexamethylindeno(5,6-c)pyran), Ethylene brassylate (1,4-dioxacycloheptadecane-5-17-dione), Habanolide (oxacyclohexadecen-2-one), Muscenone (3-methylcyclopentadecenone), Velvione (5-cyclohexadecen-1-one), Sylkolide(2-(3,5-dimethylhex-3-en-2-yloxy)-2-methylpropyl cyclopropanecarboxylate) Ambrettolide (oxacycloheptadec-10-en-2-one), Velvione (5-cyclohexadecen-1-one), Fixolide (6-acetyl-1,1,2,4,4,7-hexamethyl-1,2,3,4-tetra-hydronaphthalene).
- Powdery materials, for example Coumarin (2h-1-Benzopyran-2-one), Vanillin (4-Hydroxy-3-methoxybenzaldehyde), Ethyl Vanillin (3-Ethoxy-4-hydroxybenzaldehyde), Ethyl Maltol (3-Hydroxy-2-ethyl-4H-pyran-4-one).
- Other materials, for example Hedione (Methyl 3-oxo-2-pentylcyclopentaneacetate), Dihydro Myrcenol (2,6-Dimethyl-7-octen-2-ol), Linalyl Acetate (3,7-Dimethyl-1,6-octadien-3-yl acetate), Agrumex (2-tert-Butylcyclohexyl acetate), Galbanone (1-(5,5(3,3)-Dimethylcyclohex-1-en-1-yl)pent-4-en-1-one), Allyl Amyl Glycolate (2-Propenyl 2(3)-methylbutoxyacetate), Citronellol (3,7-Dimethyl-6-octen-1-ol), Geranium Oil, Pomarose (5,6,7-Trimethylocta-2,5-dien-4-one.

Furthermore, the perfume ingredient can be selected from diluents and other auxiliary agents commonly used in the art such as preservatives and the like. Such perfume ingredients are described in standard perfumery text books and references such as "Perfume and Flavour Chemicals", S. Arctander, Allured Publishing Corporation, 1994, IL, USA, and later editions thereof, which are incorporated herein by reference.

As adjuvants or excipients that can be employed in perfume compositions according to the invention, there can be mentioned any of those conventional ingredients, which are employed in perfume compositions for reasons other than, or not specifically related to, their odour characteristics. For example, an adjuvant or excipient may be an ingredient that acts as an aid to processing a perfume ingredient or ingredients, or a perfume composition containing said ingredient(s), or it may improve handling or storage of a perfume ingredient or perfume composition containing same. It might also be an ingredient that provides additional benefits such as imparting colour or texture. It might also be an ingredient that imparts light resistance or chemical stability to one or more ingredients contained in a perfume ingredient or perfume composition containing same. A detailed description of the nature and type of such materials that can be employed in perfume compositions containing same cannot be exhaustive, but it has to be mentioned that said ingredients are well known to a person skilled in the art. Examples include solvents and co-solvents; surfactants and emulsifiers; viscosity and rheology modifiers; thickening and gelling agents; preservative materials; pigments, dyestuffs and colouring matters; extenders, fillers and reinforcing agents; stabilisers against the detrimental effects of heat and light, bulking agents, acidulants, buffering agents and antioxidants. Any one or more of the perfume ingredients or adjuvants or excipients employed in a perfume composition according to the present invention can be formulated in a delivery vehicle if desired to provide a particular perfumery or functional effect, such as alteration of the spatio-temporal odour profile of the perfume composition. Delivery vehicles may include microcapsules, or other solid support onto which one or more perfume ingredients or adjuvants may be chemically or physically bound. Still further, one or more perfume ingredients or adjuvants may be dissolved or dispersed in a matrix material, which serves to control the rate at which said ingredient or ingredients emanates therefrom. In yet an alternative embodiment, one or more ingredients or adjuvants may be supported on a porous substrate, such as a cyclodextrin or a zeolite or other inorganic material. In still further embodiments, one or more perfume ingredients may be provided in the form of a so-called pro-perfume or perfume precursor, which can be activated under certain conditions such as moisture, heat or light to release the perfume ingredient in a controlled manner. Having regard to the foregoing, it will be appreciated that a perfume composition may be at least partly in solid form, in gel form, in foam form and/or liquid form. If it is present in solid form, it then it may take the form of granules, powders or tablets.

The perfume compositions according to the present invention need not be limited only to the aforementioned perfumer ingredients. The perfume compositions may contain one or more additional fragrance ingredients that are particularly harmonious in combination with the perfume mixture. These perfume ingredients may include any of the perfumery essential oils, alcohols, aldehydes, ketones, ethers, acetals, esters, lactones, macrocycles and heterocycles commonly used in perfumery, as well as any excipients or adjuvants conventionally used in conjunction with such perfumery ingredients for example, solvents,

The perfume mixture and perfume compositions containing same are useful in perfumery, and in particular to impart, enhance or improve a pleasant odour impression or to mask, reduce or eliminate an unpleasant odour impression, and such uses form additional aspects of the invention.

The unpleasant odour that is required to be reduced or eliminated may emanate from a consumer product into which the perfume mixture or perfume composition is incorporated, or the unpleasant odour may emanate from a situs, such as the human or animal body, or an inanimate surface, onto which the consumer product is intended to be applied in order to treat an odour.

The perfume mixtures and compositions in accordance with the invention may be employed in widely varying amounts in perfume compositions of the invention depending upon the particular olfactive effect a skilled perfumer wants to achieve. Owing to its high impact, it can be used in small quantities to achieve an olfactive effect in an economical fashion. Alternatively, because of its fresh, diffusive, transparent and easily harmonizing hedonics, it can be used in relatively high amounts to act as a harmonizer in perfume compositions across a variety of olfactive directions. Typically, the perfume mixture can be employed in widely varying amounts ranging from 0.0001 to 90 wt% of a perfume composition, preferably 0.001 to 50 wt%, more preferably 0.01 to 20 wt% and most preferably 0.1 to 10 wt%.

The third aspect of the invention is concerned with consumer products containing a perfume mixture or a perfume composition according to the first or second aspect of the invention.

The perfume mixture may be present in the consumer product in an amount ranging from about 0.0001 wt% to about 30 wt% based on the total weight of the consumer product. However, these values are given only by way of example, since the experienced perfumer may also achieve effects or may create novel accords with lower or higher concentrations.

The consumer product may be a personal care product, for example perfume extracts, eau de perfumes, eau de toilettes, aftershaves, eau de colognes, pre-shave products, splash colognes, perfumed freshening wipes, body care products, soap, liquid body wash, hair care products (e.g. shampoos, conditioners), deodorants, antiperspirants, hand creams and lotions, foot creams and lotions, hair removal creams and lotions, aftershave creams and lotions, tanning creams and lotions, and decorative cosmetic products (e.g. make-up).

The consumer product may be a cleaning product, for example, acidic, alkaline, and neutral cleaners, fabric fresheners, ironing aids, liquid detergents, fabric softeners, washing soaps, washing tablets, disinfectants (e.g. surface disinfectants), air fresheners, aerosol sprays, waxes and polishes.

The consumer product may be a homeware product, for example, candles, lamp oils, incense sticks, insecticides, repellents, and propellants.

The consumer product may be a household care product, for example a textile treatment product, an ironing aid, a cleaning cloth, a laundry detergent, a cleaning product, in particular, for hard and/or soft surfaces, a household cleaner, a care product, a wash care product, a laundry care product (for example selected from detergents, fabric conditioners, scent boosters, dryer sheets and fabric rinse products for treating fabric and applying fragrance thereto), a room fragrancer, and air freshener, a conditioner, a colorant, a fabric conditioner, a conditioning substrate, a pharmaceutical, a crop protection product, a polish, a food, a cosmetic product, a fertilizer, a building material, an adhesive, a bleach, a decalcifier, floorcare product, cookercare product, leathercare product or furniture care product, a scourer, a disinfectant, a fragrancer, a mould remover and/or a precursor of the aforementioned products.

The consumer product may be a cleaning product, such as:
- Toilet cleaners or lavatory cleaners, in other words, products for cleaning lavatory bowls and urinals, these products being supplied preferably in the form of powders, blocks, tablets or liquids, preferably gels. Besides other typical ingredients such as surfactants, they generally include organic acids e.g., citric acid and/or lactic acid) or sodium hydrogen sulfate, amidosulfuric acid or phosphoric acid for removing limescale or urine scale;
- Pipe-cleaning products or drain cleaners. These are typically strongly alkaline products which serve in general to remove pipe blockages comprising organic materials-such as hair, fat, food residues, soap deposits, and the like. Additions of Al powder or Zn powder may serve for the formation of H2 gas with an effervescence effect. Possible ingredients are commonly alkalis, alkaline salts, oxidizing agents, and neutral salts. Supply forms in powder form preferably also include sodium nitrate and sodium chloride. Pipe-cleaning products in liquid form may preferably also include hypochlorite. There are also enzyme-based drain cleaners as well. Acidic products are likewise possible;
- Universal or all-purpose or general-purpose cleaners. These are cleaners which can be used universally for all hard surfaces in the household and in commerce that can be wiped down wet or damp. Generally speaking, they are neutral or slightly alkaline or slightly acidic products, especially liquid products. All-purpose or general-purpose cleaners generally contain surfactants, builders, solvents and hydrotropes, dyes, preservatives, and the like; All-purpose cleaners with special disinfectant properties. They additionally include active antimicrobial ingredients (e.g., aldehydes, alcohols, quaternary ammonium compounds, amphoteric surfactants, triclosan and the like);

- Sanitary cleaners. These are products for cleaning in bath and toilet. The alkaline sanitary cleaners are used preferably for removing fatty soiling, whereas the acidic sanitary cleaners are employed in particular, for removing limescale. Sanitary cleaners advantageously also have a considerable disinfectant action, particularly the strongly alkaline sanitary cleaners that contain chlorine;
- Oven cleaners or grill cleaners which may be supplied in the form of gels or foam sprays. They generally serve for removing burnt-on or carbonized food residues. Oven cleaners are preferably given a strongly alkaline formulation using, for example, sodium hydroxide, sodium metasilicate, 2-aminoethanol. In addition, they generally contain anionic and/or nonionic surfactants, water-soluble solvents, and, in some cases, thickeners such as polycarboxylates and carboxymethylcellulose;
- Glass cleaners and window cleaners. These products serve preferably to remove dirt, especially greasy dirt, from glass surfaces. Preferably they contain compounds such as anionic and/or nonionic surfactants (in particular, up to 5% by weight), ammonia and/or ethanolamine (in particular, up to 1% by weight), ethanol and/or 2-propanol, glycol ethers (in particular, 10-30% by weight), water, preservatives, dyes, anti-misting agents and the like.

The consumer product may be a cosmetic product, for example (a) cosmetic skincare products, especially bath products, skin washing and cleansing products, skincare products, eye makeup, lip care products, nail care products, intimate care products, foot care products; or (b) cosmetic products with specific effects, especially sunscreens, tanning products, de-pigmenting products, deodorants, antiperspirants, hair removers, shaving products, perfumes; (c) cosmetic dental-care products, especially dental and oral care products, tooth care products, cleaners for dental prostheses, adhesives for dental prostheses; or (d) cosmetic hair care products, especially hair shampoos, hair care products, hair setting products, hair-shaping products, and hair colouring products.

The invention in its fourth aspect is concerned with a method of preparing the perfume mixture according to the first aspect of the invention.

In US patent 7,550,417 the starting material in the preparation of the ingredient Ambermax ^{™} is iso-longifolanone. Iso-longifolanone is available as a mixture of two enantiomers, namely the (-)-iso longifolanone and (+)-iso longifolanone.

The applicant has now surprisingly found that a perfume mixture prepared from iso-longifolanone that is enantio-enriched in (-)-iso longifolanone contains a higher proportion of desirable isomers and a relative paucity of isomers that display a weak or undesirable odour, and as such, such mixture is more carbon efficient and therefore sustainable.

In more particular embodiments, the (-)-iso longifolanone is in the form of a mixture of isomers with an enantiomeric excess of about 20% or more, for example 40 % or more, for example 60 % or more.

In a particular embodiment of the invention, a method of preparing the perfume mixture comprises the steps of:
- Providing iso-longifolanone in a form that is enriched in its (-)-iso longifolanone isomer; for example in an enantiomeric excess of at least about 20% ee or above, for example of about 40% ee or above, or of about 60% ee or above;
- Epimerizing (-)-iso longifolanone at the 6'-position using a base to provide an equilibrium mixture of 6'R and 6'S diasteromers, wherein the 6'R diastereomer is the major diastereomer;
- Performing a Wittig olefination on the (-)-iso longifolanone diasteromeric mixture to form Ethylidene Tetramethyl Tricyclo Undecane (ETTU) according to formula VII with concomitant inversion of the stereochemistry at the 6-position such that the 6'R ETTU diastereomer is the major diastereomer;
- Performing a Prins reaction on the ETTU diastereomeric mixture with formaldehyde or paraformaldehyde to provide a perfume mixture comprising the compounds of formula I and formula II.

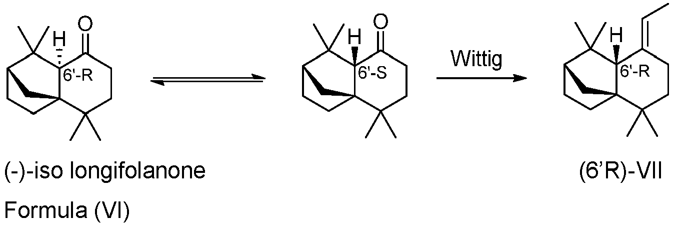

The selection of the aforementioned enantio-enriched (-)-iso longifolanone as a starting material is an important step in the process of preparing the perfume mixture according to the invention.

Accordingly, the use of enantio-enriched (-)-iso longifolanone as a starting material in the preparation of a perfume mixture according to the first aspect of the invention forms an additional aspect of the invention.

After an exhaustive study of the complex stereochemistry of the perfume mixture and its methods of preparation, the applicant found that the hydrogen at the 6'-position on the (-)-iso longifolanone ring system could be epimerized to form a diasteromeric pair of reactive intermediates (the 6'S and 6'R (-)-iso longifolanone mixture). The equilibrium distribution of reactive intermediates was such that the 6'R diastereomer was the major diastereomer, which at first sight appeared problematic as the 6'S diastereomer is a favoured isomer in terms of its olfactory performance. However, subsequent Wittig olefination reaction surprisingly proceeded with inversion of the stereochemistry of at the 6'-position of the ring system.

Without intending to be bound by any particular theory, the applicant believes that the (-)-iso longifolanone starting material 6'-position diastereoisomeric ratio has no influence on Ambermax 6'-position diastereochemistry. (-)-Iso longifolanone 6'-position stereocenter is epimerized at the Wittig stage, such that the 6'S diastereomer reacts significantly faster than the 6'R diastereomer. C6' configuration inversion is the result, which alters the distribution of ETTU diastereomers, such that the 6'R diastereomer is overwhelming the major diastereomer.

So despite the foregoing, the selection of enantio-enriched (-)-iso longifolanone as a starting material was surprisingly advantageous, leading to much higher level of desirable isomers in the perfume mixture than heretofore possible.

In a particular embodiment of the invention, the Wittig olefination can be carried out using triphenyl phosphonium salt, for example fluoride, chloride, bromide, iodide, acetate, perchlorate, tetrafluoroborate, hydroxide, phenolate phosphate, carbonate, mesylate, sulfonate, nitrate, and lithium, sodium or potassium alkoxide base, for example methoxide, ethoxyde, isopropoxide, or tert-butoxide.

In a particular embodiment of the invention, the Wittig olefination can be carried out in an aromatic, non-polar solvent, for example benzene, toluene, ethylbenzene, xylene, naphthalene, and mixtures thereof. By using such solvents, it is possible to significantly increase to overall concentration of the reaction mixture. For example, by using xylene, the concentration of the reaction mixture can be increased up to 4 times in comparison to cyclohexane. This allows for a solvent reduction of up to 75%, thereby reducing costs, waste and improving overall productivity.

In a particular embodiment of the invention, the Wittig olefination can be carried out at enhanced temperatures, that is at temperatures above 30 °C, for example in a range between 30 °C and 120°C, for example in a range between 50°C and 100 °C, for example at 80 °C, depending on the solvent used. An increase in temperature can lead to a significant reduction of the reaction time in comparison to the reaction at room temperature, and therefore to enhanced productivity.

In a particular embodiment the ETTU intermediate is obtained as a mixture of diasteroisomers in a ratio >9:1, with the 6'R diastereomer being the major diastereomer.

The ETTU intermediate is converted to a perfume mixture in accordance with the first aspect of the invention via a Prins reaction, with conservation of the stereochemistry at the 6'-position of the ring system.

In particular embodiments of the invention, the Prins reaction can be carried out using solid paraformaldehyde or aqueous formaldehyde.

In a particular embodiment of the invention, aqueous formaldehyde is used in the process. This allows for an easier handling on industrial scale since liquids are generally simpler to load into reaction vessels. In addition, the use of aqueous formaldehyde results in a cleaner reaction profile, particularly by reducing the content of low-odor-value components represented by Formula III, IV, and V. Specifically, the levels of products corresponding to Formula IV and V drops from 2 - 3 % to below 0.5% levels when aqueous formaldehyde is employed.

In a particular embodiment of the invention, solid paraformaldehyde is used in the process. Solid paraformaldehyde allows for a higher reaction concentration compared to aqueous formaldehyde, leading to reduced production costs and improved productivity. Additionally, the use of solid paraformaldehyde is associated with enhanced olfactory quality of the final product compared to using commercial aqueous formaldehyde solutions. While it is known that neat reactions with solid paraformaldehyde yield higher levels of low-odor-value components corresponding to Formulas III, IV, and V, it has been discovered that combining solid paraformaldehyde with a protic additive effectively reduces the content of these components to below 1%. Protic additives may include water, methanol, ethanol, isopropanol, butanol, or mixtures of the foregoing.

The prior art process uses water as protic additive. However, the applicant found that the reaction is preferably carried out in the presence of a protic additive consisting of a mixture of polar protic organic solvent and water. More particularly, the polar protic organic solvent is a lower alcohol, and more particularly methanol.

By the use of a mixture of polar protic organic solvent and water as protic additive, the formation of undesired formates, which negatively affect the olfactive quality of the desired product, is reduced. In comparison with the use of water as the only protic additive, the content of formates can be reduced by a factor of 10 by using a mixture of water and methanol as protic additive.

The invention is now further described with reference to the following non-limiting examples. Variations and modifications as will be readily apparent to those skilled in the art are intended to be within the scope of the present invention as defined in and by the appended claims.

### Example 1: Effect of paraformaldehyde on product formation

The Ambermax product contained the following compounds:
(1'R, 2S, 8'S)-I and (1'S, 2R, 8'R)-I: ¹H NMR (C₆D₆) δ: 3.43 (dd, *J*=10.0, 9.0 Hz, 1H), 3.34 (dd, *J*=10.0, 6.7 Hz, 1H), 2.85-2.96 (m, 1H), 1.72-1.81 (m, 3H), 1.59-1.66 (m, 2H), 1.31-1.45 (m, 3H), 1.17-1.22 (m, 1H), 1.19 (s, 3H), 1.18 (s, 3H), 1.13 (dd, *J*=9.5, 1.6 Hz, 1H), 1.06-1.13 (m, 1H), 0.93 (s, 3H), 0.88 (d, *J*=6.7 Hz, 3H), 0.87 (s, 3H) ppm. ¹³C NMR (C₆D₆) δ: 149.2 (s), 125.3 (s), 65.7 (t), 57.4 (s), 49.8 (d), 43.0 (s), 38.0 (d), 36.4 (t), 34.1 (t), 31.3 (s), 29.5 (t), 27.8 (q), 26.5 (q), 26.3 (q), 25.3 (t), 24.5 (q), 20.8 (t), 15.7 (q) ppm.
(1'R, 2R, 8'S)-I and (1'S, 2S, 8'R)-I:
   ¹H NMR (C₆D₆) δ: 3.47 (dd, *J*=10.1, 8.2 Hz, 1H), 3.36 (dd, *J*=10.1, 7.3 Hz, 1H), 2.93 (m, 1H), 1.81-1.89 (m, 1H), 1.73-1.79 (m, 1H), 1.67-1.73 (m, 1H), 1.57-1.65 (m, 2H), 1.31-1.44 (m, 3H), 1.28 (s, 3H), 1.17 (ddd, *J*=12.9, 6.3, 1.3 Hz, 1H), 1.11 (dd, *J*=9.8, 1.9 Hz, 1H), 1.08 (s, 3H), 1.01-1.07 (m, 1H), 0.92 (s, 3H), 0.88 (d, *J*=6.9 Hz, 3H), 0.84 (s, 3H) ppm. ¹³C NMR (C₆D₆) δ: 149.7 (s), 125.1(s), 66.0 (t), 57.4 (s), 49.6 (d), 43.0 (s), 38.0 (d), 36.4 (t), 34.0 (t), 31.1 (s), 29.7 (t), 27.4 (q), 27.0 (q), 26.6 (q), 25.4 (t), 24.3 (q), 20.9 (t), 15.3 (q) ppm.
(1'R, 2R, 6'S, 8'R)-II and (1'S, 2S, 6'R, 8'S)-II:
   ¹H NMR (C₆D₆) δ: 5.35 (dt, *J*=5.7, 2.5 Hz, 1H), 3.34-3.39 (m, 1H), 3.21-3.27 (m, 1H), 2.39 (sxt, *J*=6.5 Hz, 1H), 2.11 (m, 1H), 1.91 (br dd, *J*=17.4, 4.7 Hz, 1H), 1.64-1.73 (m, 2H), 1.51 (ddd, J=17.4, 5.8, 2.0 Hz, 1H), 1.26-1.44 (m, 3H), 1.09-1.11 (m, 1H), 1.14-1.23 (m, 1H), 1.08 (d, *J*=6.9 Hz, 3H), 1.08 (s, 3H), 1.00 (s, 3H), 0.89 (s, 3H), 0.83 (s, 3H) ppm. ¹³C NMR (C₆D₆) δ: 140.8 (s), 122.0 (d), 69.1 (t), 55.7 (d), 55.3 (s), 51.4 (d), 39.6 (t), 39.0 (d), 37.9 (s), 36.2 (t), 33.8 (q), 32.7 (s), 27.0 (t), 24.9 (q), 24.2 (q), 23.9 (q), 22.8 (t), 16.0 (q) ppm.

The content of compounds IV and/or V depends on the formaldehyde used in the ETTU conversion step.

### a) Solid Paraformaldehyde, no solvent:

Solid paraformaldehyde (2.58 g, 128mol%) was added to ETTU Formula VII (16 g). The reaction was heated at 180°C for 6 hours. The reaction mixture was cooled down to room temperature, yielding Ambermax crude product containing 2.8% of formate impurities (compounds of Formula IV and Formula V).

### b) Solid Paraformaldehyde, with water/MeOH as protic additive:

Solid paraformaldehyde (2.58 g, 128mol%) was added to ETTU Formula VII (16 g). Water and methanol (1:2 by weight) were added to provide a concentration of 366 g

Formaldehyde / L solvent. The reaction was heated at 180°C for 6 hours. The reaction mixture was concentrated under vacuum to remove water and methanol, yielding Ambermax crude product containing 0.2% of formate impurities (compounds of Formula IV and Formula V).

### c) Aqueous formaldehyde

Aqueous formaldehyde 37wt% (7.0 g, 128mol%) was added to ETTU Formula VII (16 g).

The reaction was heated at 180°C for 6 hours. The reaction mixture was concentrated under vacuum to remove water, yielding Ambermax crude product containing 1.1% of formate impurities (compounds of Formula IV and Formula V).

### Example 2: Isolation and characterisation of Ambermax compounds:

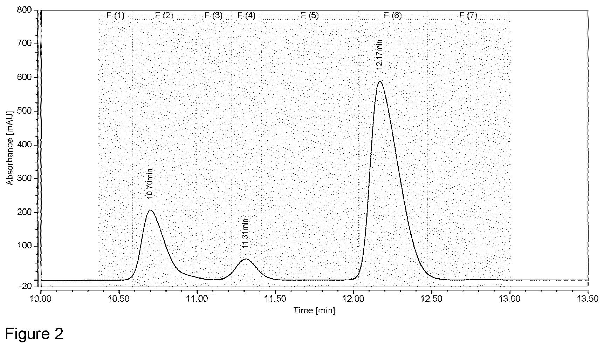

The mixture of Ambermax compounds (10mg/ml in acetonitrile, 4µl injection volume) was prefractionated by non-chiral reversed-phase HPLC with a Dionex Ultimate 3000, equipped with an UV detector at 200nm and a fraction collector AFC-3000. Separation was performed on an Ascentis^{®} Express C18 column (Merck P/N 53829-U, length 150mm, inner diameter 4.6mm, 2.7µm particle size and 90Å pore size) with isocratic elution using a mobile phase composed of water and acetonitrile (25:75, v/v) at a flow rate of 1ml/min and a column temperature of 25°C. The retention times of the collected fractions with the peaks of interest were 10.70min for fraction F2, 11.31min for fraction F4 and 12.17min for fraction F6 (see Figure 2).

The analytical purity check of the non-chiral reversed-phase HPLC fractions F2, F4 and F6 was run on a Thermo Scientific TRACE 1300 Series gas chromatograph and TriPlus 100 LS autosampler equipped with a cool on column injector, a flame ionization detector (FID) and a non-chiral capillary column VF-WAXms (Agilent P/N CP9210, polyethylene glycol stationary phase, 30m length, 0.32mm internal diameter, 0.5µm film thickness). Hydrogen was employed as carrier gas at a constant pressure of 60kPa. The oven temperature was programmed from 35°C with 2-min hold to 250°C at 3°C/min with a 10-min final temperature hold. The FID temperature was set at 270°C and the gas flow rates were air 350ml/min, hydrogen 35ml/min and nitrogen as make-up 40ml/min.

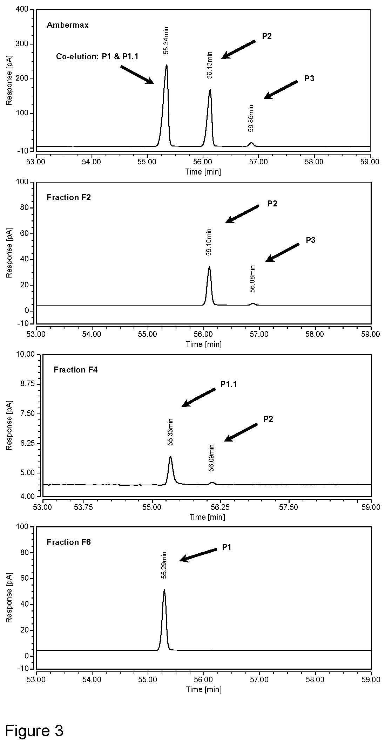

GC-FID chromatograms (see Figure 3) of Ambermax (top) and the non-chiral reversed-phase HPLC fraction F2 (second top), F4 (third top) and F6). Fraction F2 contained peak P2 as major constituent (tR 56.10min) and peak P3 as minor constituent (tR 56.88min), fraction F4 contained peak P1.1 as major constituent (tR 55.33min) and peak P2 as minor constituent (tR 56.09min), and fraction F6 contained peak P1 as major constituent (tR 55.29min).

To obtain sufficient quantities of the purified diastereoisomers P1, P1.1 and P2, 143 injections of the non-chiral reversed-phase HPLC fraction F2 and F6, and 194 injections of the non-chiral reversed-phase HPLC fraction F4 were collected. Subsequently, the combined collections of fractions F2, F4 and F6 were concentrated in a rotary evaporator to dryness, resolved in hexane and underwent an additional purification step by chiral HPLC with a Dionex Ultimate 3000, equipped with an UV detector at 200nm, a Jasco OR-4090 optical rotation detector (ORD) and a fraction collector AFC-3000. Separation was performed by 2µl injections of the hexane solutions (concentration between 1.3 and 10mg/ml) on a Regis (R,R) WHELK-O^{®}1 column (BGB Analytik P/N 1-780222-300, length 150mm, inner diameter 4.6mm, 3.5µm particle size and 100Å pore size) with isocratic elution using a mobile phase composed of hexane and isopropanol (95:5, v/v) at a flow rate of 1ml/min and a column temperature of 15°C. The combined fractions of the Ambermax stereoisomers with the indicated number of runs were concentrated in a rotary evaporator to dryness and resolved in methyl tert-butyl ether (MtBE). The concentrations of the MtBE stock solutions were determined by chiral GC-FID with external calibration. The chiral GC-FID method is described in the section below.

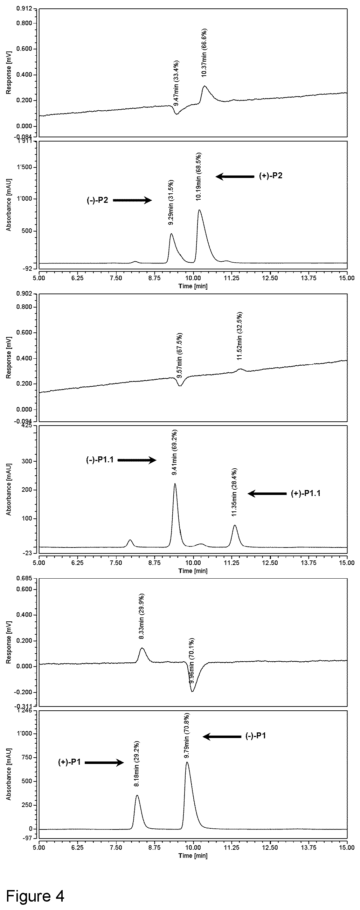

In Figure 4 the chiral HPLC chromatograms of the fractions F2 (top) with the enantiomeric pair (-)-P2 and (+)-P2, F4 (middle) with the enantiomeric pair (-)-P1.1 and (+)-P1.1, and F6 (bottom) with the enantiomeric pair (+)-P1 and (-)-P1 with the optical rotation of the Ambermax enantiomers are depicted. The top chromatogram is the ORD chromatogram, and the bottom chromatogram is the UV detection at 200nm, respectively. As these two detectors are connected sequentially, a slight retention time shift was observed.

For all Ambermax stereoisomers the olfactory purity of the MtBE stock solutions was assessed by chiral GC sniffing technique. These chiral GC sniffing analyses were performed on a Thermo Scientific TRACE 1300 Series gas chromatograph and TriPlus 100 LS autosampler equipped with a split injector using a flame ionization detector (FID) and a sniffing port (Givaudan in-house product). The column effluent was splitted 1:1 between the FID and the sniffing port using an inert glass Y-splitter and deactivated fused silica capillaries (0.5m length, 0.25mm internal diameter, BGB Analytik) of equal lengths. The transfer line to the sniffing port was heated to 250°C throughout the GC run. The separations were performed on a Hydrodex beta-3P column (Macherey-Nagel P/N 723358.25, 25m length, 0.25mm internal diameter). Hydrogen was employed as carrier gas at a constant flow of 1.5ml/min. The inlet temperature was set at 230°C with a split ratio of 20:1. The oven temperature was programmed from 50°C with 2-min hold to 230°C at 2°C/min with a 10-min final temperature hold. The FID temperature was set at 250°C and the gas flow rates were air 350ml/min, hydrogen 35ml/min and nitrogen as make-up 40ml/min.

If the main peak of the MtBE stock solution was odorless during the olfactory purity assessment, the concentration of the stock solution was used to determine the GC odor detection threshold (GTH) and the value was reported as greater than (GTH > x ng).

For all Ambermax stereoisomers where the olfactory purity of the sample could be confirmed by chiral GC sniffing analysis and the main constituent was the sample odor-vector, the GTH determinations were performed by non-chiral gas chromatography-olfactometry as described in Flachsmann et al. (2024), Ambrox through the Looking Glass: Chemoenzymatic Synthesis and GC-Olfactometric Analysis of 15 Ambrox Stereoisomers, Helvetica Chimica Acta, Volume 107, Issue 7.

In Table 1 the olfactory purity checks and GTH of the isolated Ambermax compounds are summarized.

**Table 1:**

| **Compound** | **Peak Annotation Chiral HPLC** | **Olfactory Purity Check by Chiral GC Assessed by 3 Trained Expert Assessors** | **GTH (ng)** |
|---|---|---|---|
| (1'R, 2S, 8'S)-I | (-)-P1 | olfactorily pure | 0.003 |
| (1'S, 2R, 8'R)-1 | (+)-P1 | olfactorily not pure sample contained (-)-P1 as odor-dominant trace constituent | > 83 |
| (1'R, 2R, 8'S)-I | (-)-P1.1 | olfactorily not pure sample contained (-)-P1 and (+)-P2 as odor-dominant minor constituents | > 16 |
| (1'S, 2S, 8'R)-I | (+)-P1.1 | olfactorily not pure sample contained (-)-P1 and (+)-P2 as odor-dominant minor constituents | > 9 |
| (1'R, 2R, 6'S, 8'R)-II | (-)-P2 | olfactorily not pure sample contained (-)-P1 and (+)-P2 as odor-dominant trace constituents | > 51 |
| (1'S, 2S, 6'R, 8'S)-II | (+)-P2 | olfactorily pure | 0.0038 |

### Example 3: Odorant receptor activation by different Ambermax compounds

The human odorant receptor OR7C1 is specifically activated by molecules with an ambery odour character.

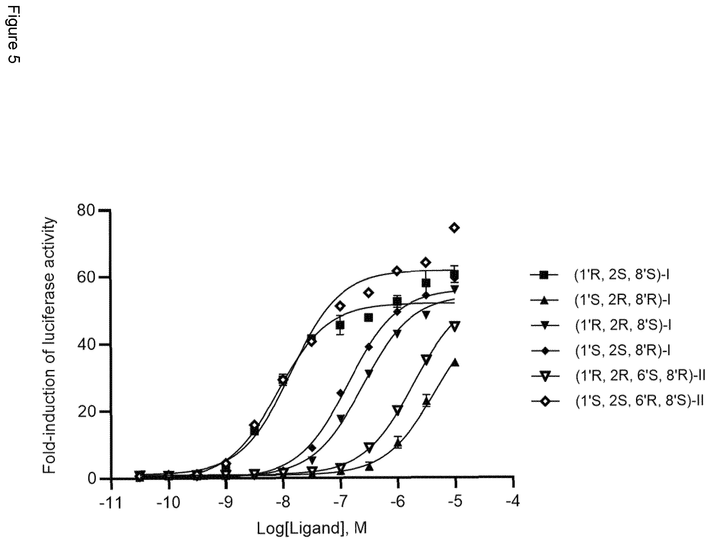

The potency of the Ambermax isomers was therefore determined by measuring the *in vitro* activation of the OR7C1(S99G,V126I,S210P,V247L) haplotype variant, which is more sensitive to ambery molecules than the standard OR7C1 variant reported in GenBank. As shown in Fig. 5, compound (1'R, 2S, 8'S)-I and compound (1'S, 2S, 6'R, 8'S)-II are the most potent compounds.

It was found that compounds (1'R, 2S, 8'S)-I and (1'S, 2S, 6'R, 8'S)-II are olfactorily pure, while the other compounds contain said most potent compounds in traces. Therefore, the activation of OR7C1(S99G,V126I,S210P,V247L) by the other compounds is probably caused by minor amounts of (1'R, 2S, 8'S)-I and compound (1'S, 2S, 6'R, 8'S)-II rather than by the compounds as such.

The coding DNA sequence for human OR7C1(S99G,V126I,S210P,V247L) with an optimized C-terminal domain (DNA sequences SEQ ID NO: 1 and amino acid sequence SEQ ID NO: 2) was synthesized by a DNA synthesis service provider (BioCat GmbH, Germany) and inserted into pcDNA3.1(+) using BamHI and Notl restriction sites (Invitrogen, MA, USA) downstream of the CMV promoter sequence (SEQ ID NO: 3) and before the bgh terminator sequence (SEQ ID NO: 4). The synthetic OR nucleotide sequence further contained at thier 5'-end a Kozak-sequence (GCCACC) and a nucleotide sequence encoding a signal peptide (mmLucy-FLAG-rho) (SEQ ID NO: 5 and 6). This plasmid thus contains a constitutively expressed OR7C1 gene.

Expression of the OR7C1(S99G,V126I,S210P,V247L) gene was performed in HEK293T cells that had been stably transfected with a DNA sequence coding for functional variants of the human RTP1S (V227I) (SEQ ID NO: 7 and 8) and RTP2(L220R) (SEQ ID NO: 9 and 10). These cells were seeded into polyethyleneimine coated 96-well plates (100µl/well) at a density of 10,000 cells/well and grown at 37°C in presence of 5% CO2 for 24 h. Growth medium was DMEM containing 9% fetal bovine serum (FBS) and penicillin / streptomycin (GibcoTM, ThermoFisher Scientific, MA, USA)

0.6 µg of the OR7C1(S99G,V126I,S210P,V247L) expression plasmid, 1µg of empty pcDNA3.1(+) vector, and 1 µg of pGL4.29 (Promega) harbouring the CRE-inducible luciferase gene were pipetted into a microtube. The DNA mix was then diluted in 0.25 ml OptiMEM medium (GibcoTM, ThermoFisher Scientific, MA, USA) containing 5ul of P3000 reagent. In parallel, 5 µl Lipofectamine 3000 (Invitrogen) were diluted in 0.25 ml OptiMEM medium. After 5 min pre-incubation, the two mixtures were combined to prepare the transfection mixture, which was incubated for an additional 25 min.

50 µl of growth medium was replaced with fresh DMEM containing 9% fetal bovine serum (FBS) and penicillin / streptomycin. The transfection mixture was diluted in 5 ml OptiMEM medium and 50 µl of the diluted mixture was added per well (final total volume is 150 µl). Cells were further incubated for 24 h at 37°C in the presence of 5% CO2 to allow for DNA uptake and expression of OR7C1(S99G,V126I,S210P,V247L).

OR7C1(S99G,V126I,S210P,V247L) activation by the test compounds was measured by removal of 100 µl growth medium and addition of 50 µl DMEM containing 9% FBS, penicillin / streptomycin, 2% DMSO, and different concentrations of the test substances. After incubation for 4.5 h, the cells were lysed using 20 µl passive lysis buffer (Promega) and the luciferase signal, which is induced based on OR-dependent cAMP production, was measured.

### Sequences:

OR7C1(S99G,V126I,S210P,V247L) DNA sequence with an optimized C-terminus DNA se-quence (SEQ ID NO 1): 
ATGGAAACAGGAAATCAAACACATGCCCAAGAATTTCTCCTCCTGGGATTTTCAGCAA
CGTCAGAGATTCAGTTCATTCTCTTTGGGCTGTTCCTCTCCATGTACCTAGTCACTTT
CACCGGGAACCTGCTCATCATCCTGGCCATATGCTCAGACTCCCACCTCCACACCCC
CATGTACTTCTTCCTCTCCAACCTGTCTTTTGCTGACCTCTGTTTTACCTCCACGACT
GTCCCAAAGATGTTACTGAATATACTGACACAGAACAAATTCATAACATATGCAGGCT
GTCTCGGTCAGATTTTTTTTTCACTTCATTTGGATGCCTGGACAATTTACTCTTGACC
GTGATGGCCTATGACCGCTTCGTGGCCATCTGTCACCCCCTGCACTATACGGTCATC
ATGAACCCCCAGCTCTGTGGACTGCTGGTTCTGGGGTCCTGGTGCATCAGTGTCAT
GGGTTCCCTGCTCGAGACCTTGACTGTTTTGAGGCTGTCCTTCTGCACCGAAATGGA
AATTCCACACTTTTTTTGTGATCTACTTGAAGTCCTGAAGCTCGCCTGTTCTGACACC
TTCATTAATAACGTGGTGATATACTTTGCAACTGGCGTCCTGGGTGTGATTCCCTTCA
 
CTGGAATATTTTTCTCTTACTATAAAATTGTTTTCTCTATACTGAGGATTTCCTCAGCT
GGGAGAAAGCACAAAGCGTTTTCCACCTGTGGTTCCCACCTCTCACTGGTCACCTTG
TTCTATGGCACGGGCTTTGGGGTCTATCTCAGTTCTGCAGCCACACCATCTTCTAGG
ACAAGTCTGGTGGCCTCAGTGATGTACACCATGGTCACCCCCATGCTGAACCCCTTC
ATCTACAGCCTGAGGAACAAAGAAGTTAAAAAGGCCATAAAGAGGTTGTTCAAGAGA
AAGTGCTGCAGGAGAAGGTGA
OR7C1(S99G,V126I,S210P,V247L) amino acid sequence with an optimized C-terminus ami-no acid sequence (SEQ ID NO 2): 
METGNQTHAQEFLLLGFSATSEIQFILFGLFLSMYLVTFTGNLLIILAICSDSHLHTPMYFFL
SNLSFADLCFTSTTVPKMLLNILTQNKFITYAGCLGQIFFFTSFGCLDNLLLTVMAYDRFVA
ICHPLHYTVIMNPQLCGLLVLGSWCISVMGSLLETLTVLRLSFCTEMEIPHFFCDLLEVLKL
ACSDTFINNVVIYFATGVLGVIPFTGIFFSYYKIVFSILRISSAGRKHKAFSTCGSHLSLVTLF
YGTGFGVYLSSAATPSSRTSLVASVMYTMVTPMLNPFIYSLRNKEVKKAIKRLFKRKCCR
RR
CMV Promoter DNA sequence (SEQ ID NO 3) 
GTTGACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGCTGA
CCGCCCAACGACCCCCGCCCATTGACGTCAATAATGACGTATGTTCCCATAGTAACG
CCAATAGGGACTTTCCATTGACGTCAATGGGTGGAGTATTTACGGTAAACTGCCCAC
TTGGCAGTACATCAAGTGTATCATATGCCAAGTACGCCCCCTATTGACGTCAATGAC
GGTAAATGGCCCGCCTGGCATTATGCCCAGTACATGACCTTATGGGACTTTCCTACT
TGGCAGTACATCTACGTATTAGTCATCGCTATTACCATGGTGATGCGGTTTTGGCAGT
ACATCAATGGGCGTGGATAGCGGTTTGACTCACGGGGATTTCCAAGTCTCCACCCCA
TTGACGTCAATGGGAGTTTGTTTTGGCACCAAAATCAACGGGACTTTCCAAAATGTCG
TAACAACTCCGCCCCATTGACGCAAATGGGCGGTAGGCGTGTACGGTGGGAGGTCT
ATATAAGCAGAGCTC
bgh terminator DNA sequence (SEQ ID NO 4) 
CTGTGCCTTCTAGTTGCCAGCCATCTGTTGTTTGCCCCTCCCCCGTGCCTTCCTTGA
CCCTGGAAGGTGCCACTCCCACTGTCCTTTCCTAATAAAATGAGGAAATTGCATCGC
ATTGTCTGAGTAGGTGTCATTCTATTCTGGGGGGTGGGGTGGGGCAGGACAGCAAG
GGGGAGGATTGGGAAGACAATAGCAGGCATGCTGGGGATGCGGTGGGCTCTATGG
mmLucy-FLAG-rho DNA sequence (SEQ ID NO 5) 
ATGAGCCACCAGATCCTGCTGCTCCTGGCCCTGCTGACCCTAGGCCTGGCTGATTA
CAAGGACGACGACGATAAGATCGAATTGATGAACGGGACCGAGGGCCCAAACTTCT
ACGTGCCTTTCTCCAACAAGACGGGCGTGGTGGAATTC
mmLucy-FLAG-rho amino acid sequence (SEQ ID NO 6)
   MSHQILLLLALLTLGLADYKDDDDKIELMNGTEGPNFYVPFSNKTGVVEF
RTP1S(V227I) DNA sequence (SEQ ID NO 7) 
ATGTGTAAAAGCGTGACCACAGATGAGTGGAAGAAAGTCTTCTATGAGAAGATGGAG
GAGGCAAAGCCGGCTGACAGCTGGGACCTCATCATAGACCCCAACCTCAAGCACAA
TGTGCTGAGCCCTGGTTGGAAGCAGTACCTGGAATTGCATGCTTCAGGCAGGTTCCA
CTGCTCCTGGTGCTGGCACACCTGGCAGTCGCCCTACGTGGTCATCCTCTTCCACAT
GTTCCTGGACCGCGCCCAGCGGGCGGGCTCGGTGCGCATGCGCGTCTTCAAGCAG
CTGTGCTATGAGTGCGGCACGGCGCGGCTGGACGAGTCCAGCATGCTGGAGGAGA
ACATCGAGGGCCTGGTGGACAACCTCATCACCAGCCTGCGCGAGCAGTGCTACGGC
GAGCGTGGCGGCCAGTACCGCATCCACGTGGCCAGCCGCCAGGACAACCGGCGGC
ACCGCGGAGAGTTCTGCGAGGCCTGCCAGGAGGGCATCGTGCACTGGAAGCCCAG
CGAGAAGCTGCTGGAGGAGGAGGCGACCACCTACACCTTCTCCCGGGCGCCCAGC
CCCACCAAGTCGCAGGACCAGACGGGCTCAGGCTGGAACTTCTGCTCTATCCCCTG
GTGCTTGTTTTGGGCCACGGTCCTGCTGCTGATCATCTACCTGCAGTTCTCTTTCCGT
AGCTCCATCTAA
RTP1S(V227I) amino acid sequence (SEQ ID NO 8) 
MCKSVTTDEWKKVFYEKMEEAKPADSWDLIIDPNLKHNVLSPGWKQYLELHASGRFHC
SWCWHTWQSPYVVILFHMFLDRAQRAGSVRMRVFKQLCYECGTARLDESSMLEENIEG
LVDNLITSLREQCYGERGGQYRIHVASRQDNRRHRGEFCEACQEGIVHWKPSEKLLEEE
ATTYTFSRAPSPTKSQDQTGSGWNFCSIPWCLFWATVLLLIIYLQFSFRSSI
RTP2(L220R) DNA sequence (SEQ ID NO 9) 
ATGTGTACCAGCTTGACCACTTGTGAGTGGAAGAAAGTCTTCTATGAGAAGATGGAG
GTGGCAAAGCCAGCGGACAGCTGGGAGCTCATCATAGACCCCAACCTCAAGCCCAG
TGAGCTGGCCCCTGGCTGGAAGCAGTACCTGGAGCAGCACGCCTCAGGCAGGTTC
CACTGCTCCTGGTGCTGGCACACCTGGCAGTCTGCCCATGTGGTCATCCTCTTCCAC
ATGTTCCTGGACCGCGCCCAGCGGGCGGGCTCGGTGCGCATGCGCGTCTTCAAGC
AGCTGTGCTATGAGTGCGGCACGGCGCGGCTGGACGAGTCCAGCATGCTGGAGGA
GAACATCGAGGGCCTGGTGGACAACCTCATCACCAGCCTGCGCGAGCAGTGCTACG
 
AGGAGGATGGTGGCCAGTACCGCATCCACGTGGCCAGCCGCCCGGACAGCGGGCC
GCATCGTGCAGAGTTCTGTGAGGCCTGCCAGGAGGGCATCGTTCACTGGAAGCCCA
GCGAGAAGCTGCTGGAGGAGGAGGTGACCACCTACACCTCTGAAGCCTCCAAGCCG
AGGGCCCAGGCGGGATCCGGCTACAACTTCTTGTCTCTTCGCTGGTGCCTCTTCTG
GGCCTCTCTCTGCCTGCTCGTTGTTTACCTGCAGTTCTCCTTCCGCAGTCCTGCCTT
CTTTTAG
RTP2(L220R) amino acid sequence (SEQ ID NO 10)

MCTSLTTCEWKKVFYEKMEVAKPADSWELIIDPNLKPSELAPGWKQYLEQHASGRFHCS
WCWHTWQSAHVVILFHMFLDRAQRAGSVRMRVFKQLCYECGTARLDESSMLEENIEGL
VDNLITSLREQCYEEDGGQYRIHVASRPDSGPHRAEFCEACQEGIVHWKPSEKLLEEEV
TTYTSEASKPRAQAGSGYNFLSLRWCLFWASLCLLVVYLQFSFRSPAFF

## Claims

1. A perfume mixture comprising a compound according to formula I or formula II 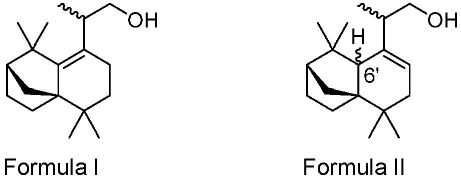 wherein the perfume mixture is free, or substantially free, of a compound according to formula III 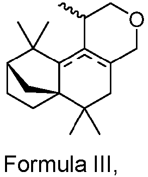 and wherein the wavy bond in the compound of formula I, II and/or III indicates an unspecified configuration at the attached carbon atom, respectively, and the dotted lines together with the solid lines 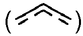 in the compound of formula III indicate one single and one double bond.

2. The perfume mixture according to claim 1 that is free or substantially free of a compound according to formula IV or formula V 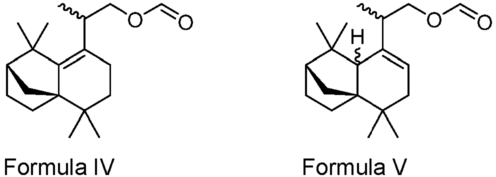 wherein the wavy bond in the compound of formula IV and/or V indicates an unspecified configuration at the attached carbon atom, respectively.

3. The perfume mixture according to claim 1 or claim 2, wherein a compound of formula II is enriched in the 6'R diastereomer relative to its 6'S diastereomer.

4. The perfume mixture according to any of the preceding claims wherein the diastereomeric ratio 6'R diastereomer:6'S diastereomer in a compound of formula II is at least 9:1.

5. The perfume mixture according to any of the preceding claims wherein the compounds of formula I and of formula II are in an enantiomeric excess of about 20% ee or above, for example of about 40% ee or above, or of about 60% ee or above.

6. A perfume composition comprising the perfume mixture according to any of the preceding claims and at least one additional perfume ingredient.

7. A consumer product comprising a perfume mixture according to any of the preceding claims.

8. A method of preparing a perfume mixture according to claim 1 comprising the steps of
- Providing (-)-iso longifolanone (compound of formula VI) in an enantiomeric excess of at least about 20% ee or above, for example of about 40% ee or above, or of about 60% ee or above 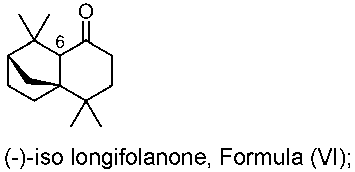
- Epimerizing (-)-iso longifolanone at the 6'-position using a base to provide an equilibrium mixture of 6'R and 6'S diasteromers, wherein the 6'R diastereomer is the major diastereomer;
- Performing a Wittig olefination on the (-)-iso longifolanone diasteromeric mixture to form Ethylidene Tetramethyl Tricyclo Undecane (ETTU) according to formula VII with concomitant inversion of the stereochemistry at the 6-position such that the 6'R ETTU diastereomer is the major diastereomer; 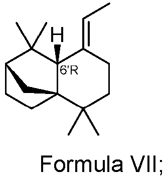
- Performing a Prins reaction on the ETTU diastereomeric mixture with formaldehyde or paraformaldehyde to provide a perfume mixture comprising the compounds of formula I and formula II.

9. The method according to claim 8 wherein the Wittig reaction is carried out using triphenyl phosphonium bromide or triphenyl phosphonium chloride and sodium or potassium tert-butoxide.

10. The method according to claim 8 or claim 9 wherein the reaction with formaldehyde or paraformaldehyde is carried out in the presence of a protic additive, for example an alcohol, preferably methanol.

11. The method according to any of the claims 8 through 10, wherein the paraformaldehyde or formaldehyde is in an excess relative to the protic additive /water mixture.

12. The method according to claim 11, wherein the paraformaldehyde or formaldehyde is in a 1.2 excess at least relative to the protic additive /water mixture.
